(19) Europäisches Patentamt
European Patent Office
Office européen des brevets

(11) EP 4 740 935 A1

(12) **EUROPEAN PATENT APPLICATION**

(43) Date of publication:
**13.05.2026 Bulletin 2026/20**

(21) Application number: **24212203.4**

(22) Date of filing: **11.11.2024**

(51) International Patent Classification (IPC):
***A61K 9/00*** *(2006.01)* ***A61K 9/08*** *(2006.01)*
***A61K 31/7088*** *(2006.01)* ***A61K 47/02*** *(2006.01)*

(52) Cooperative Patent Classification (CPC):
**A61K 9/0078; A61K 9/08; A61K 31/7088; A61K 47/02**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicants:
- **RNATICS GmbH**
  **82152 Planegg (DE)**
- **Technische Universität München, in Vertretung des Freistaats Bayern**
  **80333 München (DE)**

(72) Inventors:
- **Frischmuth, Thomas.**
  **22607 Hamburg (DE)**
- **Engelhardt, Stefan.**
  **80802 München (DE)**
- **Beck, Christina.**
  **82152 Martinsried (DE)**
- **Schmidt, Johannes.**
  **82152 Martinsried (DE)**

(74) Representative: **Weickmann & Weickmann PartmbB**
**Postfach 860 820**
**81635 München (DE)**

Remarks:
The complete document including Reference Table(s) and the Sequence Listing(s) can be downloaded from the EPO website

(54) **FORMULATION OF OLIGONUCLEOTIDE DRUGS FOR INHALATION**

(57) The present invention relates to a formulation for inhalation comprising an active agent which is an oligonucleotide drug and further constituents. The formulation is stable when stored for extended time as well as during aerosolization and physiologically well tolerated.

EP 4 740 935 A1

## Description

### Field of the Invention

The present invention relates to a formulation for inhalation comprising an active agent which is an oligonucleotide drug and further constituents. The formulation is stable when stored for extended time as well as during aerosolization and physiologically well tolerated.

### Background of the Invention

In the treatment of respiratory tract disorders, drugs are frequently administered by inhalation. For this purpose, stable formulations having a long shelf-life are needed. On the other hand, the formulations should be physiologically tolerable.

WO 2021/205032, the content of which is herein incorporated by reference, discloses inhibitors of miR-21 which may be administered by inhalation. These inhibitors may comprise an oligonucleotide drug, e.g., an antisense molecule directed against microRNA-21 (miR-21) conjugated to a targeting moiety for delivery to lung fibroblasts and/or lung macrophages. No specific formulations for inhalation are described.

The publication by Beck et al. (Nature Communications 14 (2023): 4564; doi.org/10.1038/s41467-023-40185-1), the content of which is herein incorporated by reference, discloses a trimannose-conjugated antisense inhibitor of microRNA-21, designated RCS-21, as a therapeutic agent against hyperinflammation and fibrosis. No specific formulations for inhalation are described.

It was an objective of the present invention to provide a stable and physiologically tolerable formulation for inhalation comprising as an active agent an oligonucleotide drug, particularly a conjugate of an oligonucleotide with a targeting moiety.

### Summary of the Invention

A first aspect of the present invention is a formulation for inhalation comprising and particularly consisting of

(i) an active agent which is an oligonucleotide drug;
(ii) NaCl;
(iii) water; and
(iv) optionally a buffer.

A further aspect of the present invention is a formulation for inhalation as described above for use in medicine, particularly in human medicine.

Still a further aspect of the present invention is a method of treating a subject in need thereof, particularly a human subject, comprising administering to said subject a therapeutically effective amount of a formulation by inhalation, wherein the formulation consists of or comprises

(i) an active agent which is an oligonucleotide drug;
(ii) NaCl;
(iii) water; and
(iv) optionally a buffer.

In certain embodiments, the oligonucleotide drug is a conjugate comprising an oligonucleotide moiety and a targeting moiety for delivering the oligonucleotide moiety to a predetermined target cell, e.g., a macrophage. In certain embodiments, the targeting moiety is a carbohydrate moiety, e.g., a trimannose moiety.

In even more particular embodiments, the oligonucleotide drug is compound RCS-21 or a physiologically acceptable salt thereof:

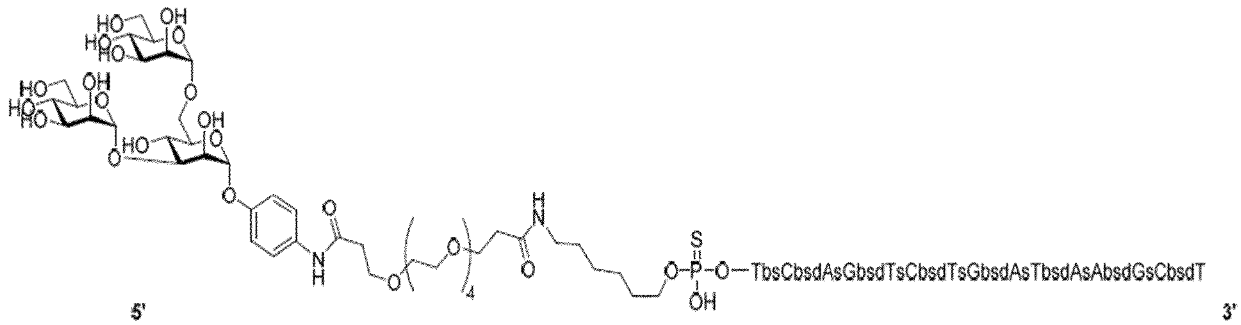

wherein the capital letters *T*, *C, A* and *G* in the oligonucleotide moiety represent nucleotide building blocks comprising the nucleobases thymine, 5-methyl cytosine, adenine, and guanine, respectively,

the lower-case letter *s* represents a phosphorothioate internucleosidic bond between two nucleotide building blocks,

the lower-case letter *b* after a capital letter denotes a locked nucleotide building block having a 2'-O-$CH_2$-4' bridge, and

the lower-case letter *d* before a capital letter denotes a 2'-deoxyribonucleotide building block.

**Detailed description**

The present inventors have prepared aqueous liquid formulations comprising the oligonucleotide drug RCS-21 and tested their stability under different storage conditions. They found that the oligonucleotide drug remains stable after storage over an extended time period at -20°C, +5°C, +25°C and +40°C as determined by assessment of clarity and color of the solution as well as concentration, purity and impurity profile of the active agent. Further, the inventors found that the formulations are very well suited for aerosolization, e.g., using a mesh or a jet nebulizer, generating a wet aerosol without foam formation and allowing the administration of a high dose rate. In addition, the inventors found, that the drug substance in the aerosolized liquid formulations is stable during the aerosolization process and stays intact. Furthermore, the formulations have a high safety and tolerability in experimental animals.

In certain embodiments, the formulation of the present invention is stable when stored for at least 3 months, for at least 6 months, for at least 9 months, for at least 12 months, or for at least 18 months at -20°C.

In certain embodiments, the formulation of the present invention is stable when stored for at least 1 month, for at least 3 months, or for at least 12 months at +5°C.

In certain embodiments, the formulation of the present invention is stable when stored for at least 1 month, for at least 3 months, for at least 6 months or for at least 12 months at +25°C.

In certain embodiments, the formulation of the present invention is stable when stored for at least 1 month at +40°C.

In certain embodiments, stability is determined by evaluation of appearance of the test solution (clarity and color) according to visual inspection.

In certain embodiments, stability is determined by evaluation of the concentration of the oligonucleotide drug, e.g. by determining absorbance at 260 nm (A260) according to UV/Vis spectrophotometric measurement in an aqueous solution.

The heterocyclic bases of DNA and RNA have the attitude to absorb in the ultraviolet region (~260 nm) of the electromagnetic spectrum. Therefore, the absorbance (A) of DNA and RNA can be measured at a wavelength of 260 nm and the concentration (c) determined using the Lambert-Beer law in conjunction with the molar extinction coefficient ($\varepsilon$) and the optical path length (l):

$$A = \varepsilon \times c \times l$$

The UV measurement is performed by diluting an oligonucleotide sample with deionized water or matrix diluent in the linear absorption range from 0.3 Abs to 3.0 Abs. The dilution is then filled into a Quartz or disposable plastic semi-micro cuvette and prepared at the appropriate position in the UV/Vis spectrophotometer. The UV detector is set to 260 nm to detect the absorption of the oligonucleotide, from which the concentration can then be calculated.

In certain embodiments, stability is determined by evaluation of the purity and impurities of the oligonucleotide drug, e.g. by ion-pair reversed-phase high performance liquid chromatography combined with electron spray mass spectrometry (IP-RP-HPLC-ESI-MS) according to method KU-PRA-SOP-042. In brief, a sample of the oligonucleotide

drug is applied to a chromatography column and separated by gradient elution using a buffer system. After elution, a UV detector at 260 nm records the eluting compounds. Absolute retention times (RT), relative retention times (RRT) to the full-length product (FLP) and peak resolutions (Rs) are calculated. Relative peak areas of the parent compound and its related sequences are determined for purity analysis.

In particular embodiments, the formulation is stable when:

(i) the formulation is a clear solution (e.g. comparable to water);
(ii) the formulation is a colorless solution;
(iii) the concentration of the oligonucleotide drug is within a range of +/- 10%, particularly of +/- 5% based on the initial concentration at begin of storage (t = 0);
(iv) the purity of the oligonucleotide drug is within a range of +/- 5%, particularly of +/- 2% based on the initial purity at begin of storage (t = 0); and/or (v) the pH is in the range of pH 5-8.

In certain embodiments, the formulation of the present invention is an aqueous liquid, particularly an aqueous solution, more particularly a clear and colorless aqueous solution. In certain embodiments, the formulation is free of any organic solvent.

For inhalation, the formulation of the present invention is aerosolized to provide a wet aerosol, by suitable means, e.g. by using a nebulizer.

In certain embodiments, the formulation is a liquid ready-to-use formulation, which is stored in a pre-formulated state, e.g., as an aqueous liquid formulation. The liquid ready-to-use formulation may be prepared dissolving the oligonucleotide drug in an aqueous liquid, e.g., an isotonic NaCl solution and filling it under suitable, e.g., sterile conditions into a container. The container is stored until use.

In certain embodiments, the formulation is a reconstitutable formulation, wherein the oligonucleotide drug is stored in a dry state, e.g. as a lyophilizate, in a container and is reconstituted by adding a reconstitution liquid, e.g., an isotonic NaCl solution before use.

The formulation of the present invention comprises an oligonucleotide drug as an active agent. The term "oligonucleotide drug" relates to an oligonucleotide, e.g., single-stranded or double-stranded oligonucleotide, which may be conjugated to a heterologous moiety. In certain embodiments, the oligonucleotide comprises at least about 5 nucleotide building blocks and up to 100 or more nucleotide building blocks. In further embodiments, the oligonucleotide comprises between about 5 to about 50 nucleotide building blocks, between about 10 and about 30 nucleotide building blocks, and particularly between about 12 and about 25 nucleotide building blocks. The oligonucleotide may comprise at least one nucleotide building block selected from a ribonucleotide building block, a deoxyribonucleotide building block, a modified nucleotide building block, or any combination thereof. In certain embodiments, the oligonucleotide comprises at least one modified nucleotide building block.

In certain embodiments, the modified nucleotide building block may be a 2'-modified ribonucleotide wherein the 2'-OH substituent of ribose moiety is replaced by halo, e.g., F, $-C_1-C_5$ alkyl, $-O-C_1-C_5$ alkyl, e.g. $-OCH_3$, $-S-C_1-C_5$ alkyl, $-C_2-C_5$ alkenyl, $-O-C_2-C_5$ alkenyl, $-S-C_2-C_5$ alkenyl, $-C_2-C_5$ alkynyl, $-O-C_2-C_5$ alkynyl, $-S-C_2-C_5$ alkynyl, amino including mono- or disubstituted amino, e.g., $-C_1-C_5$ alkyl, $-C_2-C_5$ alkenyl or $-C_2-C_5$ alkynyl substituted amino wherein each alkyl, alkenyl or alkynyl group is optionally substituted with OH, halo, cycloalkyl, cycloalkenyl, (hetero)aryl, e.g. phenyl, O-alkyl, S-alkyl, and/or amino.

In certain embodiments, the modified nucleotide building block is a bridged, e.g. a 2'-4'-bridged nucleotide building block, wherein the bridge typically has a length of 2-5 atoms, particularly 2-3 atoms including C-atoms and optionally heteroatoms such as O, N or S. In a particular embodiment, the modified building block is a locked nucleotide building block having a $2'-O-CH_2-4'$ bridge.

In certain embodiments, the modified nucleotide building block is a building block wherein the ring of the ribose moiety is modified, e.g. a morpholino building block, a thio-ribose building block, a 6-membered pyranose building block or a peptidic nucleic acid (PNA) building block.

In certain embodiments, the modified nucleotide building block comprises a modified internucleosidic linkage wherein the phosphoester group connecting adjacent building blocks is replaced by modified internucleosidic linkage, e.g. a phosphorothioate linkage, an alkyl phosphonate, e.g. methyl phosphonate linkage, and a borano phosphate linkage.

In certain embodiments, the oligonucleotide drug comprises an antisense molecule, i.e., a single stranded oligonucleotide comprising about 12-25 nucleotide building blocks. In certain embodiments, the oligonucleotide includes or consists of modified nucleotide building blocks, e.g. locked nucleotide building blocks. In particular embodiments, the oligonucleotide consists of locked nucleotide building blocks and deoxyribonucleotide building blocks.

In certain embodiments, the oligonucleotide drug is a microRNA antagonist, which is capable of binding to a microRNA and thereby inhibiting its activity. In particular embodiments, the oligonucleotide drug is a miR-21 antagonist.

In very particular embodiments, the oligonucleotide drug comprises an oligonucleotide having the nucleotide sequence (SEQ ID NO.1):

5'-TCAGTCTGATAAGCT-3'

In even more particular embodiments, the oligonucleotide drug comprises an oligonucleotide having the nucleotide sequence (SEQ ID NO. 2):

5'-TCaGtCtGaTaAgCt -3'

wherein a capital letter represents a locked nucleic building block, a lower-case letter represents a 2'-deoxyribonucleotide building block, every capital C denotes 5-methyl cytosine, and all internucleosidic linkages are phosphorothioate linkages.

In certain embodiments, the oligonucleotide is conjugated to a heterologous moiety, e.g. a targeting moiety for directing the oligonucleotide drug to a target cell, e.g. a macrophage. In certain embodiments, the heterologous moiety is a peptide, lipid or carbohydrate moiety. In particular embodiments, the heterologous moiety is a carbohydrate moiety, more particularly a trimannose moiety.

The oligonucleotide may be conjugated to the heterologous moiety via a linker. In certain embodiments, the linker is attached to the 5'- end of the oligonucleotide. In certain embodiments, the linker is a polyethylene glycol containing linker.

In most particular embodiments, the oligonucleotide drug is RCS-21.

The formulation of the present invention comprises the oligonucleotide drug in an amount which allows a therapeutically effective delivery. In certain embodiments, the oligonucleotide drug is present in an amount of about 1 mg/ml to about 100 mg/ml or about 5 mg/ml to about 50 mg/ml, particularly about 10 mg/ml to about 25 mg/ml and more particularly about 13 mg/ml to about 22 mg/ml, e.g. about 14 or about 20 mg/ml based on the volume of liquid, e.g., in a ready-to-use formulation or in a reconstituted formulation.

The formulation of the present invention further comprises NaCl. In certain embodiments, NaCl is present in an amount of about 7 mg/ml to about 11 mg/ml particularly about 8 mg/ml to about 10 mg/ml and more particularly about 9 mg/ml (based on the volume of liquid, e.g., in a ready-to-use formulation or in a reconstituted formulation.

In certain embodiments, the formulation of the present invention has a pH of about 5 to about 8, particularly about 5.0 to about 8.0, more particularly about 6 and even more particularly about 6.0. If necessary, the pH of the formulation may be adjusted, e.g., with HCl or NaOH or a buffer, e.g., a phosphate buffer, to a range of about 5.0 to about 8.0, particularly to about 6.0.

In certain embodiments, a formulation of the present invention e.g., a ready-to-use formulation or a reconstitutable formulation, consists of the oligonucleotide drug, NaCl, i.e., sodium (as ion), chloride (as ion) and water. A ready-to-use formulation consists of the oligonucleotide drug, NaCl, i.e., sodium (as ion), chloride (as ion) and water wherein all components may be provided in a single container. A reconstitutable formulation consists of the oligonucleotide drug, NaCl, i.e., sodium (as ion), chloride (as ion) and water wherein the oligonucleotide drug in a dry state, e.g., as a lyophilizate, may be provided in a first container and the reconstitution liquid may be provided in a second container. In certain embodiments, NaCl is provided with the reconstitution liquid.

In certain embodiments, the formulation of the present invention further comprises a buffer for pH adjustment, e.g., a phosphate buffer, e.g., sodium phosphate buffer, a potassium phosphate buffer or a phosphate-based saline buffer. In certain embodiments, the buffer is present in an amount of providing a formulation with a calculated osmolality of about 260 to about 360 mOsmol/L, particularly about 308 mOsmol/L, which is the calculated osmolality of a physiological sodium chloride solution. In certain embodiments, the formulation comprises 1 mM to about 30 mM potassium chloride and 1 mM to about 20 mM potassium phosphate monobasic. In certain embodiments 0.1 mM to about 2 mM sodium chloride and 5 mM to about 100 mM sodium phosphate dibasic is added. Particularly, the pH of the buffer is between about 7 ot about 8, more particularly about 7.0 to about 8.0.

In certain embodiments, the formulation of the present invention, e.g., a ready-to-use formulation or a reconstitutable formulation, consists of the oligonucleotide drug, NaCl, i.e., sodium (as ion), chloride (as ion), Na-phosphate, i.e., sodium (as ion) and phosphate (including all forms of non-protonated, partially protonated, and protonated phosphate), and water. A ready-to-use formulation of the present invention consists of the oligonucleotide drug, NaCl, i.e., sodium (as ion), chloride (as ion), the buffer components, e.g., sodium and phosphate (as ions) and water wherein all components may be provided in a single container. A reconstitutable formulation consists of the oligonucleotide drug, NaCl, i.e., sodium (as ion), chloride (as ion), the buffer components, e.g., sodium and phosphate (as ions) and water. The oligonucleotide drug in a dry state, e.g., as a lyophilizate, may be provided in a first container and the reconstitution liquid may be provided in a second container. In certain embodiments, NaCl and/or the buffer components are provided with the reconstitution liquid.

In particular embodiments, the formulation of the present invention is free from any surfactant. In further particular embodiments, the formulation of the present invention is free from any preservative.

In certain embodiments, the formulation is a single-dose formulation. In those embodiments, the formulation may be provided in a container comprising a single inhalation dose of the oligonucleotide drug. In further embodiments, the formulation is a multi-dose formulation. In those embodiments, the formulation may be provided in a container comprising multiple, e.g. 2, 3, 4, 5, 6, 7, 8, 9 or 10 or even more inhalation doses of the oligonucleotide drug.

The formulation of the present invention may be provided in a container suitable for pharmaceutical use, e.g., a glass or plastic container provided with a stopper and a top cap.

In certain embodiments, the formulation is present in a volume of about 0.5 ml to about 50 ml, particularly about 1 ml to about 10 ml, and more particularly about 3-5 ml.

In certain embodiments, the formulation is administered daily or even 2- or 3-times daily. In certain embodiments, the formulation is administered weekly, twice weekly or every second day. In certain embodiments, the formulation is administered bi-weekly, monthly, bi-monthly, tri-monthly or every 6 months.

An inhalation dose for a subject to be treated will depend on the type and severity of the disease. For human patients, an inhalation dose is typically in the range of about 0.1 mg to about 1000 mg, e.g. about 1 mg to about 200 mg.

In certain embodiments, the formulation is sterile. Sterility may be provided by sterile filtration, aseptic filling and/or utilization of sterilized, e.g., autoclaved equipment.

The formulation of the present invention is intended for use in medicine, particularly in human medicine. In certain embodiments, the formulation is for administration by nasal inhalation. In further embodiments, the formulation is for administration by oral inhalation.

For administration, the formulation is aerosolized to provide an aerosolized formulation which is a wet aerosol. The aerosol is administered to a subject, particularly a human subject, in a therapeutically effective amount by inhalation, e.g., by nasal inhalation or by oral inhalation. Typically, the aerosol has a droplet size of about 1-10 μm, in particular of about 3-5 μm. The aerosolized formulation may be administered to the lungs, bronchi and/or airways.

Aerosolization may comprise pressurization and/or nebulization, using a pressurized pack or a nebulizer, e.g., a mesh nebulizer or a jet nebulizer. In certain embodiments, a propellant may be added to the formulation, e.g., dichlorodifluoromethane, trichlorofluoromethane, dichlorotetrafluoroethane, carbon dioxide or other suitable gases. In certain embodiments, capsules and cartridges for use in an inhaler or insufflator may be formulated. Formulations and methods for modulating the size of droplets using nebulizer devices to target specific portions of the respiratory tract and lungs are well known to those skilled in the art.

The formulation is particularly suitable for the treatment of a disorder of the respiratory tract, particularly for the treatment of a disorder of the lower respiratory tract, more particularly for the treatment of a pulmonary disorder.

In particular embodiments, the formulation is suitable for the treatment of a disorder which is associated with, caused by and/or accompanied with pathological fibroblast and/or macrophage activity in the respiratory tract, e.g., in the lungs.

Specifically, the formulation of the present invention is suitable for the treatment of a disorder selected from pulmonary fibrosis, pneumonia, ARDS, COPD, a viral or bacterial pulmonary infection, e.g. an infection by a coronavirus such as SARS-CoV-2, or influenza virus, or Respiratory syncytial virus (RSV), or a parainfluenza virus, or a rhinovirus, or an adenovirus, or a metapneumovirus, a cancer of the respiratory tract, e.g., a lung cancer, and an inflammatory lung disease.

The formulation may be administered as a monotherapy or, simultaneously or sequentially, as a combination therapy with a further drug. For example, the composition provided herein will be administered in the treatment in combination with a pharmaceutical composition for the treatment of viral and/or bacterial infection(s) and anti-inflammatory treatments. Such a treatment may include but is not limited to an antiviral agent, e.g., Remdesivir, a beta-adrenergic receptor agonist, e.g., Salbutamol and/or a glucocorticoid, e.g., Dexamethasone.

Further, the present invention shall be explained in more detail by the following Examples.

## Examples

### Example 1 - Stability of a ready-to-use formulation comprising 14 mg/mL RCS-21

1.1. Formulation

The composition of a ready-to-use formulation comprising 14 mg/ml oligonucleotide drug RCS-21 is summarized in Table 1:

**Table 1. Description of RCS-21 14 mg/mL**

| INGREDIENT | QUANTITY/ML | QUANTITY/VIAL |
|---|---|---|
| RCS-21 | 14 mg | 63 mg |
| Sodium chloride | 9 mg | 40.5 mg |
| 0.1M HCl | For adjustment of pH to the target value 6.0 at T=23-25°C (acceptable range 5.0+7.0) | |
| 0.1M NaOH | For adjustment of pH to the target value 6.0 at T=23-25°C (acceptable range 5.0+7.0) | |
| Water for injections (WFI) | q.s. to 1.0 mL | q.s. to 4.5 mL |

(continued)

| INGREDIENT | QUANTITY/ML | QUANTITY/VIAL |
|---|---|---|
| Filling volume (overfill included) | | 4.5 mL |
| Nominal volume | | 4.5 mL |

4.5 mL of the formulation were filled into an ISO 6R glass vial comprising a rubber stopper (diameter 20 mm) and an aluminum seal top cap (diameter 20 mm).

1.2. Storage and test conditions

The formulation was stored at the following conditions, inverted position, exposed to indoor laboratory light during the sample handling and analysis:

- - 20°C ± 5°C inverted (long-term condition)
- + 5°C ± 3°C inverted (accelerated condition)
- + 25°C ± 2°C / 60% relative humidity (RH) ± 5% RH inverted (accelerated condition)

Goal of this study was a stability assessment of a liquid formulation of the oligonucleotide drug RCS-21.
Samples were tested for:

- Appearance of solution,
- pH of solution
- Identity, purity and impurities by denaturing ion-pairing reversed-phase ultra-high-performance liquid chromatography (IP-RP-UHPLC) coupled with electrospray ionization-mass spectrometry (ESI-MS), and
- Concentration by ultraviolet (UV) assay.

For appearance testing, clarity and degree of opalescence of the formulation were compared to freshly prepared reference suspensions comprising of a mixture of hydrazine sulphate solution and hexamethylenetetramine solution at different dilutions in ultrapure water against a black background 5 min after preparation. Identical test tubes of clear, colorless, and neutral glass with a flat base were used.
For the assessment of color, a test was carried out using scaled up solutions of inorganic reagents for the 3 primary colors (Blue: cupric sulphate; Yellow: ferric chloride; Red: cobalt chloride) properly mixed to create the 5 reference color solutions being compared: Yellow, Brown, Green-Yellow, Brown-Yellow, and Red.
For the assessment of visible particle contamination, the integrity of the container and its closure were verified, all labels removed from the container and rinsed clean. Then the container was gently rotated without formation of air bubbles and the solution observed for at least 5 s against a white background. The process was repeated, and the solution was observed for at least 5 s against a black background. The whole process was thereafter repeated with inverting the container. After each observation, the presence of any particles and/or fibers was recorded.
The pH of the solution was measured according to a standard method.
For the ion-pairing reversed-phase ultra-high performance liquid chromatography (IP-RP-UHPLC) analysis was used. A sample of RCS-21 was applied to a chromatography column and separated by gradient elution using a buffer system. After elution, a UV detector at 260 nm records the eluting compounds. Absolute retention times (RT), relative retention times (RRT) to the full-length product (FLP) and peak resolutions (Rs) are calculated. Relative peak areas of the parent compound and its related sequences are determined for purity analysis.
Determination of concentration by UV measurement was performed by diluting an oligonucleotide sample with deionized water or matrix diluent in the linear absorption range from 0.3 Abs to 3.0 Abs. The dilution was then filled into a Quartz or disposable plastic semi-micro cuvette and prepared at the appropriate position in the UV/Vis spectrophotometer. The UV detector was set to 260 nm to detect the absorption of the oligonucleotide, from which the concentration was then calculated.
For release of the material at T=0 and the other pull points, the concentration of the liquid formulation (free acid form of the total oligonucleotide content) was determined by UV spectroscopy using the experimentally determined molar extinction coefficient (119691 $L \cdot mol^{-1} \cdot cm^{-1}$).
At each pull point one analysis was conducted for each storage condition.
The purity and impurities were reported according to the relative peak area-% greater 0.2% for the oligonucleotide drug in the liquid formulation. For stability analysis the peak area-% of the main compound in the analysis was compared with the peak area% at T=0 for all pull points.

1.3. Results

Test results are shown in the following Tables 2-4:

Table 2: Stability at -20°C T 0-12 months

| Test | Results T=0 | Results T=1M | Results T=3M | Results T=6M | Results T=9M | Results T=12M |
|---|---|---|---|---|---|---|
| APPEARANCE OF SOLUTION - CLARITY | Clear solution, comparable to water | Clear solution, comparable to water | Clear solution, comparable to water | Clear solution, comparable to water | Clear solution, comparable to water | Clear solution, comparable to water |
| APPEARANCE OF SOLUTION - COLOR | Colorless solution | Colorless solution | Colorless solution | Colorless solution | Colorless solution | Slightly yellowish solution, comparable to Y7 |
| pH | 5.4 | 6.1 | 6.1 | 6.0 | 6.0 | 6.1 |
| CONCENTRATION (A260) | 13.6 mg/mL | 13.5 mg/mL | 13.3 mg/mL | 13.2 mg/mL | 13.2 mg/mL | 13.1 mg/ml |
| PURITY (HPLC) | 91.1 % | 90.9 % | 89.8 % | 88.3 % | 88.0% | 90.5% |

Table 3: Stability at +5°C T 0-12 months

| Test | Results T=0 | Results T=1M | Results T=3M | Results T=12M |
|---|---|---|---|---|
| APPEARANCE OF SOLUTION - CLARITY | Clear solution, comparable to water | Clear solution, comparable to water | Clear solution, comparable to water | Clear solution, comparable to water |
| APPEARANCE OF SOLUTION - COLOR | Colorless solution | Colorless solution | Colorless solution | Slightly yellowish solution, comparable to Y7 |
| pH | 5.4 | 6.4 | 6.1 | 6.2 |
| CONCENTRATION (A260) | 13.6 mg/mL | 13.3 mg/mL | 13.5 mg/mL | 13.0 mg/ml |
| PURITY (HPLC) | 91.1 % | 90.9 % | 89.1 % | 90.4% |

Table 4: Stability at +25°C T 0-12 months

| Test | Results T=0 | Results T=1M | Results T=3M | Results T=12M |
|---|---|---|---|---|
| APPEARANCE OF SOLUTION - CLARITY | Clear solution, comparable to water | Clear solution, comparable to water | Clear solution, comparable to water | Clear solution, comparable to water |
| APPEARANCE OF SOLUTION - COLOR | Colorless solution | Colorless solution | Colorless solution | Slightly yellowish solution, comparable to Y7 |
| pH | 5.4 | 6.1 | 6.1 | 6.0 |
| CONCENTRATION (A260) | 13.6 mg/mL | 13.4 mg/mL | 13.2 mg/mL | 12.9 mg/ml |
| PURITY (HPLC) | 93.4 % | 90.6 % | 88.6 % | 86.9 % |

The above results demonstrate that the formulation is stable at all tested storage conditions.

**Example 2 - Stability of a ready-to-use formulation comprising 19.7 mg/mL RCS-21**

2.1 Formulations

Further tests were conducted with a ready-to-use formulation comprising 19.7 mg/mL RCS-21 and 0.9% (w/v) NaCl.

0.5 mL of the liquid formulation were filled into 2 mL sterile transparent type I glass vials comprising a rubber stopper and an aluminum seal top cap.

2.2 Storage and test conditions

Glass vials for storage of the liquid reference standard were stored in upright position and contain 0.5 mL of 19.7 mg/mL RSC-21 (free acid form of the total oligonucleotide content). An appropriate volume was drawn from the vial and diluted to the target concentration of 0.2 mg/mL (free acid form of the FLP content) with isotonic saline (0.9 % (w/v)) to perform the IP-RP-HPLC analysis.

The liquid formulation was stored at the following conditions, upright position, exposed to indoor laboratory light during the sample handling and analysis:

- - 20°C ± 5°C
- +25°C ± 2°C / 60% RH ± 5% RH
- + 40°C ± 2°C / 75% RH ± 5% RH

Samples were pulled at the indicated time points and tested for/with:

- Appearance
- Denaturing IP-RP-HPLC purity and impurities
- Concentration by ultraviolet (UV) assay.

2.3 Test results

Test results are shown in the following Tables 5-7:

Table 5: Stability of liquid formulation at -20°C T 0-18 months

| Test | Results T=0 | Results T=3M | Results T=6M | Results T=12M | Results T=18M |
|---|---|---|---|---|---|
| APPEARANCE OF SOLUTION - CLARITY AND COLOR | Clear and colorless solution, uniform appearance, free from visible contaminants | Clear and colorless solution, uniform appearance, free from visible contaminants | Clear and colorless solution, uniform appearance, free from visible contaminants | Clear and colorless solution, uniform appearance, free from visible contaminants | Clear and colorless solution, uniform appearance, free from visible contaminants |
| PURITY (HPLC) | 90.0 % | 90.7 % | 90.5 % | 90.9 % | 89.8 % |
| PURITY COMPARED TO T0 (%) | - | 100.8 % | 100.5 % | 101.0 % | 99.7 % |
| CONCENTRATION (A260) | - | 100.4 % | 95.8 % | 99.4 % | 100.2 % |

Table 6: Stability of liquid formulation at +25°C T 0-6 months

| Test | Results T=0 | Results T=1M | Results T=3M | Results T=6M |
|---|---|---|---|---|
| APPEARANCE OF SOLUTION - CLARITY AND COLOR | Clear and colorless solution, uniform appearance, free from visible contaminants | Clear and colorless solution, uniform appearance, free from visible contaminants | Clear and colorless solution, uniform appearance, free from visible contaminants | Clear and colorless solution, uniform appearance, free from visible contaminants |

(continued)

| Test | Results T=0 | Results T=1M | Results T=3M | Results T=6M |
|---|---|---|---|---|
| PURITY (HPLC) | 90.0 % | 90.9% | 90.9 % | 90.4% |
| PURITY COMPARED TO T0 (%) | - | 101.0% | 101.0% | 100.5% |
| CONCENTRATION (A260) | - | 100.5% | 101.9% | 97.4% |

Table 7: Stability of liquid formulation at +40°C T 0-1 months

| Test | Results T=0 | Results T=1M |
|---|---|---|
| APPEARANCE OF SOLUTION - CLARITY AND COLOR | Clear and colorless solution, uniform appearance, free from visible contaminants | Clear and colorless solution, uniform appearance, free from visible contaminants |
| PURITY (HPLC) | 90.0 % | 90.8 % |
| PURITY COMPARED TO T0 (%) | - | 100.9 % |
| CONCENTRATION (A260) | - | 100.4 % |

The above results demonstrate that the formulation is stable at all tested storage conditions.

## Example 3 - Physicochemical characterization

RCS-21 was weighed (0.3479 g and 0.8124 g) into volumetric flasks (50 mL). NaCl solution was added to approx. 45 mL and the powder was dissolved by intense shaking. Afterwards the temperature was checked, and the flasks were filled to 50 mL at 20°C. The resulting concentrations were 6.958 mg/mL and 16.248 mg/ml.

All measurements were performed in triplicate at 20 °C (± 1 °C).

### 3.1 Density

The density of the samples was measured by bending vibration with the density meter DMA 48 (Anton Paar). It was calibrated with water and air at 20°C. Samples (approx. 2 mL) were injected into the capillary with a syringe.

### 3.2 Vibro-Viscosimetry

The SV-10 Vibro Viscosimeter (Malvern Instruments) was used for determining viscosity using vibrating paddles. The instrument was calibrated with Milli-Q Water bevor use. A small volume of the sample was used to rinse the sample cell prior to the measurements. Measurement was performed with 10 mL of the respective solutions. The measured density of the samples was considered for the calculation.

### 3.3 Ubbelohde Capillary Viscosimetry

A capillary 0a (Schott) was used with the AVS/N (Schott) for capillary viscosimetry assessment. The capillary was rinsed with a small volume of the respective sample prior to the measurements. The correction of time according to the user manual as well as the capillary constant (K = 0.000498) from the certificate of analysis and the measured density were considered for the calculation.

### 3.4 Surface Tension

The surface tension was measured using a platin-iridium ring according to Du Noüy at the Tensiometer K-12 (Krüss). The surface tension of water was checked beforehand as reference. The sample glass was rinsed with a small volume of the respective sample prior to the measurements of approx. 20 mL of the samples. The density of the sample was considered for the measurement. The same sample was used three times, and the ring was annealed before each of the measurements.

3.5 pH-Value

The pH value was assessed at a pH 540 GLP from WTW using a glass electrode, which was calibrated directly before use. An aliquot of the solutions was filled into a small glass to measure pH.

3.6 Results

Table 8 summarizes the physicochemical characterization of RCS-21 solutions:

| RCS-21 concentration | Density in g/mL | Dynamic Viscosity in mPa·s | | Surface Tension in mN/m | pH |
|---|---|---|---|---|---|
| | | Vibro | Ubbelohde | | |
| 6.958 mg/mL | 1.0083 ± 0 | 1.02 ± 0.02 (0.998 - 1.032) | 1.15 ± 0.05 (1.10 - 1.21) | 67.07 ± 2.2 (64.58 - 69.56) | 6.07 ± 0.01 (6.05 - 6.07) |
| 16.248 mg/mL | 1.0121 ± 0 | 1.16 ± 0.01 (1.156-1.169) | 1.53 ± 0.02 (1.51 - 1.55) | 62.46 ± 4.6 (57.25 - 67.68) | 6.15 ± 0.02 (6.13 - 6.16) |

**Example 4 - Nebulization**

A test series (with n = 2 measurements) was carried out for different concentrations (7 mg/ml and 17.5 mg/ml) with two different jet nebulizers and two different mesh nebulizer systems. Before and after the test series, the reservoirs of the nebulizer were weighed. The amount of test substance remaining in the reservoir was determined by subtracting the post weight from the pre-weight values. For the measurements, the reservoir of the two jet nebulizers was filled with 1.2 ml solution of RCS-21 (7 mg/ml, 17.5 mg/ml) and the reservoir of the two mesh nebulizer systems was filled with 1.5 ml solution of RCS-21 (7 mg/ml, 17.5 mg/ml). The nebulizers were connected to the test setup with a connector.

After the respective nebulizer was connected to the experimental set-up, a sinus pump and an inhaler were switched on and the time until the aerosol delivery was finished was noted. The sinus pump was protected by a filter (Whatman). The nebulizability (nebulization behavior, observation of potential foam in the reservoir and the observation of correct start and stop behavior) was recorded by a camera. The nebulization time until emptying was measured with a stopwatch.

Table 9 summarizes the results of the tests regarding nebulizability of the aerosol:

| Test items | Original amount [mg] | Residual amount [mg] | Output rate [mg/sec] |
|---|---|---|---|
| Jet nebulizer 1 [7mg/ml]<br>Jet nebulizer 1 [17.5mg/ml] | 1015.2<br>1204.4 | 681.9<br>952.8 | 3.88<br>3.64 |
| Jet nebulizer 2 [7mg/ml]<br>Jet nebulizer 2 [17,5mg/ml] | 1198.3<br>1204.2 | 757.6<br>824.5 | 3.42<br>3.13 |
| Mesh nebulizer system 1 [7mg/ml]<br>Mesh nebulizer system 1 [17,5mg/ml] | 1495.4 ±9.0<br>1505.9 ±1.1 | 155.4 ±67.8<br>89.10 ±31.4 | 7.92 ±0.54<br>7.64 ±0.41 |
| Mesh nebulizer system 2 [7mg/ml]<br>Mesh nebulizer system 2 [17,5mg/ml] | 1491.8 ±6.9<br>1492.9 | 46.5 ±12.1<br>36.3 | 6.99 ±0.45<br>6.23 |

The above results demonstrate a higher output rate for the mesh nebulizer system with a lower residual amount in the reservoir at the end of the nebulization. Furthermore, surprisingly no foaming was observed with any of the used nebulizer combinations.

**Example 5 - Droplet size distribution by laser diffraction**

A test series (with n = 1 measurement) per concentration (7 mg/ml and 17.5 mg/ml) and per nebulizer (Jet nebulizer 1 and 2, Mesh nebulizer System 1 and 2) to determine the droplet size distribution by laser diffraction was conducted. For this purpose, the jet nebulizers were filled with 1.2 ml solution of RCS-21 and the mesh nebulizer systems were filled with 0.5 ml solution of RCS-21 and attached to a spectrometer. The Malvern-Spraytec-System and the

nebulizers were operated according to their instructions for use. The measurements were conducted continuously until the end of aerosol generation.

Table 10 summarizes the results of the droplet size distribution. Dv50 is the median particle diameter for a volume distribution that splits the distribution with half above and half below this diameter. GSD is the geometric standard deviation.

| Test items | Dv50 | GSD |
|---|---|---|
| Jet nebulizer 1 [7 mg/ml] | 4,8 | 2 |
| Jet nebulizer 1 [17.5 mg/ml] | 4,6 | 1,94 |
| Jet nebulizer 2 [7 mg/ml] | 3,1 | 1,8 |
| Jet nebulizer 2 [17.5 mg/ml] | 3,1 | 1,8 |
| Mesh nebulizer system 1 [7 mg/ml]<br>Mesh nebulizer system 1 [17.5 mg/ml] | 4,5<br>4,6 | 1,64<br>1,64 |
| Mesh nebulizer system 2 [7 mg/ml]<br>Mesh nebulizer system 2 [17.5 mg/ml] | 4,1<br>4,3 | 1,67<br>1,45 |

The above results demonstrate similar droplet size distribution with any of the used nebulizer combinations. Furthermore, samples were collected in a sampling vial and frozen after nebulization. Integrity tests of the drug substance were performed using the ion-pairing reversed-phase ultra-high performance liquid chromatography (IP-RP-UHPLC) method as described above. Interestingly, no decomposition of the drug substance was detected.

**Example 6 - Particle size distribution and dose rates**

The aim of the work conducted was to investigate the particle size distribution, dose and dose rate during the nebulization of RCS-21 formulation (concentration 14 mg/ml) with Jet nebulizer 2 and Mesh nebulizer system 2.

For each nebulizer, one test series (with n = 3 measurements) was carried out to determine the dose and dose rate and one test series (with n = 3 measurements) was carried out to determine the particle size distribution.

6.1 Particle size measurements

For the measurement, the test nebulizer was filled with 3 ml test and connected to the test stand via the connector. The inhaler was operated in the specified mode via the control unit and the aerosol released at the patient interface was collected on the impactor plates of an NGI impactor (operated at 30 l/min) and a back-up collection filter.

6.2 Determination of Dose and Dose rate

For the measurement to determine the dose and the dose rate, the inhaler was filled with 3 ml of the RCS-21 formulation and connected to a test bench. After approximately 3 min for the Jet nebulizer 2 and after approximately 2:25 mins for the Mesh nebulizer system 2 (approximate nebulization time for 2/3 of 1 ml of the test substance), the inhaler was turned off. Additionally, the sinus pump was paused and the collection filter 1 was exchanged with the collection filter 2. The sinus pump and the inhaler were switched on again. This procedure was repeated for collection filter 2 and collection filter 3.

300 μl of the remaining test substance in the reservoir of the Jet nebulizer 2 was collected and analyzed.

The masses collected on the collection filter were determined gravimetrically (see chapter 4.5) after re-drying and conditioning.

6.3 Results

6.3.1 Particle size distribution

**Jet nebulizer 2**

Table 11 summarizes the results of the size distribution measurements of the aerosol generated from the RCS-21 formulation with the Jet nebulizer 2. Mass median diameter (MMAD) and width (sg) of size distribution found after

nebulization are shown.

Table 11:

| Measurement | 1 | 2 | 3 | Mean value |
|---|---|---|---|---|
| MMAD (μm) | 2.47 | 2.39 | 2.56 | 2.47 +/-0.07 |
| σg | 2.23 | 2.33 | 2.11 | 2.22 +/-0.09 |
| Fine particle fraction (< 5 μm) | 80.9% | 79.6% | 80.1% | 80.1% +/-0.5% |

**Mesh nebulizer system 2**

Table 12 summarizes the results of the size distribution measurements of the aerosol generated from the RCS-21 formulation with the Mesh nebulizer system 2.

Table 12:

| Measurement | 1 | 2 | 3 | Mean value |
|---|---|---|---|---|
| MMAD (μm) | 3.12 | 2.86 | 3.22 | 3.07+/- 0.15 |
| σg | 1.59 | 1.68 | 1.67 | 1.65+/- 0.04 |
| Fine particle fraction (< 5 μm) | 81.4 | 87.9 | 77.4 | 81.2% +/-9.4% |

6.3.2 Dose and Dose Rate and Residuals (inhaler)

Table 13 shows a summary of dose and dose rate measurements for Jet nebulizer 2.

Table 13:

| | | |
|---|---|---|
| Nebulized Dose (mg RCS-21) | 28.6 | ± 0.36 |
| Inhaled Dose (mg RCS-21) | 13.5 | ± 0.19 |
| Inhaled fraction of nebulized dose | 47% | ± 1.9% |
| Inhaled fraction of nominal dose | 32% | ± 1.4% |
| Dose rate (mg RCS-21 /min) | 1.50 | ± 0.072 |
| Dose rate (percentage of nominal dose/min) | 3.6% | ± 0.17% |

Table 14 shows a summary of dose and dose rate measurements for Mesh nebulizer system 2.

Table 14:

| | | |
|---|---|---|
| Nebulized Dose (mg RCS-21) | 29.9 | ± 1.2 |
| Inhaled Dose (mg RCS-21) | 21.7 | ± 0.63 |
| Inhaled fraction of nebulized dose | 72% | ± 4.9% |
| Inhaled fraction of nominal dose | 52% | ± 2.9% |
| Dose rate (mg RCS-21 /min) | 3.10 | ± 0.245 |
| Dose rate (percentage of nominal dose/min) | 7.4% | ± 0.58% |

6.3.3 Deposition Probability as function of breathing frequency

The depositions probabilities were calculated using MPPDE 3.02 ® assuming following parameters:

FRC Volume: 3.3 l
Head Volume: 50 ml
Breathing route: Oral
Tidal Volume: 625 ml
Inspiration Fraction: 0.5
Breathing Frequency: (8, 12,15) l/min

Table 15 shows the calculated deposition probabilities for the three different breathing frequencies for the Jet nebulizer 2 for the airway areas Head, Tracheobronchial (TP) and pulmonary (P). For a breathing frequency of 12/min the sum of the deposition probabilities in the two lung aeras is 36%. Taking into account the results presented in table 16 the deposited fraction of the nominal dose can be estimated to be 8.3% for the two lung areas (TB +P).

Table 15:

| | Breathing frequency (l/min) | | |
|---|---|---|---|
| | **8** | **12** | **15** |
| **Head** | 7.4% | 9.0% | 10.1% |
| **TB** | 18.4% | 14.2% | 12.4% |
| **P** | 23.5% | 21.8% | 20.5% |

Table 16 shows the calculated deposition probabilities for the three different breathing frequencies for the Mesh nebulizer system 2. For a breathing frequency of 12/min the sum of the deposition probabilities in the two lung areas is 43%. Taking into account the results presented in table 7 the deposited fraction of the nominal dose can be estimated to be 22% for the two lung areas (TB +P).

Table 16:

| | Breathing frequency (l/min) | | |
|---|---|---|---|
| | **8** | **12** | **15** |
| **Head** | 6.0% | 7.8% | 9.0% |
| **TB** | 21.1% | 16.2% | 14.1% |
| **P** | 28.6% | 26.8% | 25.1% |

As summary the expected deposited fraction of the nominal dose in the lung for the Mesh nebulizer system 2 is by a factor 2-3 higher than for the Jet nebulizer 2.

**Example 7 - Tolerability**

Pre-clinical animal studies have shown that the formulation is well tolerated in rats following the intravenous (bolus) administration and in dogs after administration by inhalation.

In rats the formulation was administered once daily by intravenous (bolus) administration for 2 weeks, at the doses of 2.5, 10, and 25 mg/kg body weight/day. The formulation was well tolerated at the doses of 2.5 and 10 mg/kg/day. In both sexes, only at the highest dose of 25 mg/kg/day, minor clinical symptoms were observed primarily on Day 1, in the first hours after dosing. A decrease in body weight and body weight gain was also noted, in correlation with a lower food consumption. Clinical pathology parameters were affected at the highest dose which were mostly reversible. No formulation-related changes were noted in coagulation parameters.

Due to the accumulation of drug-related material in the renal tubules having caused minimal to mild (at 10 mg/kg/day) or mild to moderate (at 25 mg/kg/day) degeneration of the renal tubules, the lesions observed at these doses were considered adverse.

Based on these results, the no observed adverse effect level (NOAEL) was considered to be 2.5 mg/kg/day.

In dogs the formulation was administered as aerosol by inhalation once daily for 2 weeks at the delivered doses of 1, 2.5, and 10 mg/kg body weight/day. Administration of the formulation by inhalation was well tolerated in dogs at levels up to 10 mg/kg/day. Target organ effects were observed at levels of ≥1 mg/kg/day and consisted of increased alveolar macrophages in the lung and increased histiocytes in the tracheobronchial lymph nodes which were only partially reversible. In the absence of any degenerative changes, the microscopic findings and accompanying increased lung weight were regarded as non-adverse and attributable to storage of the test material in alveolar macrophages and regional lymph nodes/histiocytes (Levin et al., 2008; Braendli-Baiocco et al., 2017; Leqvio [inclisiran] EPAR.) Based on these results, the no-observed adverse-effect level (NOAEL) was considered to be 10 mg/kg/day.

In an additional study in dogs the effects of the formulation on arterial blood pressure, heart rate, lead II electrocardiogram (ECG) and body temperature following inhalation administration was evaluated. The formulation was administered as aerosol by inhalation once at the delivered doses of 1, 2.5, and 10 mg/kg body weight and no notable changes in heart rate, arterial blood pressure, lead II ECG intervals or QA interval considered to be test item-related were

observed. At target delivered doses of 1 and 2.5 mg/kg, no notable changes in body temperature considered to be test item-related were observed. In addition, no lead II ECG waveform morphology abnormalities were noted, there was no effect of the test item on body weight and no clinical signs were associated with administration of the formulation.

## Claims

**1.** A formulation for inhalation consisting of or comprising

(i) an active agent which is an oligonucleotide drug;
(ii) NaCl;
(iii) water; and
(iv) optionally a buffer.

**2.** The formulation of claim 1, which is a ready-to-use formulation.

**3.** The formulation of claim 1, which is a reconstitutable formulation.

**4.** The formulation of any one of claims 1-3, which is for oral inhalation.

**5.** The formulation of any one of claims 1-4, which is a single-dose formulation.

**6.** The formulation of any one of claims 1-3, wherein the oligonucleotide drug is present in an amount of about 1 mg/ml to about 100 mg/ml, about 5 mg/ml to about 50 mg/ml, about 5 mg/ml to about 20 mg/ml, about 13 mg/ml to about 15 mg/ml, or about 14 mg/ml based on the volume of liquid.

**7.** The formulation of any one of claims 1-5, wherein NaCl is present in an amount of about 7 mg/ml to about 11 mg/ml, about 8 mg/ml to about 10 mg/ml or particularly about 9 mg/ml based on the volume of liquid.

**8.** The formulation of any one of claims 1-7, wherein the pH is about 5.0 to about 8.0, particularly about 6.0.

**9.** The formulation of any one of claims 1-8, wherein the oligonucleotide drug is a miR-21 antagonist.

**10.** The formulation of any one of claims 1-9, wherein the oligonucleotide drug is RCS-21 or a physiologically acceptable salt thereof:

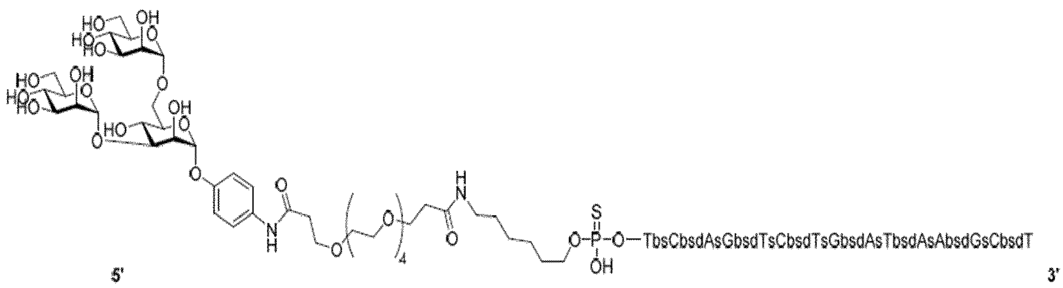

wherein the capital letters *T*, C, *A* and G in the oligonucleotide moiety represent nucleotide building blocks comprising the nucleobases thymine, 5-methyl cytosine, adenine, and guanine, respectively,
the lower-case letter s represents a phosphorothioate internucleosidic bond between two nucleotide building blocks,
the lower-case letter b after a capital letter denotes a locked nucleotide building block having a 2'-O-$CH_2$-4' bridge, and
the lower-case letter d before a capital letter denotes a 2'-deoxyribonucleotide building block.

**11.** The formulation of any one of claims 1-10, wherein the buffer is a phosphate buffer, particularly a sodium phosphate buffer.

**12.** The formulation of any one of claims 1-11, which consists of components (i), (ii), and (iii) or which consists of components (i), (ii), (iii) and (iv).

**13.** The formulation of any one of claims 1-12 for use in human medicine.

**14.** The formulation of any one of claims 1-13 for use in the treatment of a disorder selected from pneumonia, COPD, a viral infection, cancer, and an inflammatory lung disease.

**15.** A method of treating a subject in need thereof, comprising administering to said subject a therapeutically effective amount of a formulation by inhalation, wherein the formulation comprises

(i) an active agent which is an oligonucleotide;
(ii) NaCl;
(iii) water; and
(iv) optionally a buffer.

Europäisches Patentamt
European Patent Office
Office européen des brevets

# EUROPEAN SEARCH REPORT

Application Number
EP 24 21 2203

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | SEIDL LEONARDO L ET AL: "Antisense oligonucleotides and their technical suitability to nebulization", INTERNATIONAL JOURNAL OF PHARMACEUTICS, ELSEVIER, AMSTERDAM, NL, vol. 661, 25 June 2024 (2024-06-25), XP087575576, ISSN: 0378-5173, DOI: 10.1016/J.IJPHARM.2024.124390 [retrieved on 2024-06-25] * table 1; page 2, right column, middle to page 3, left column, middle * ----- | 1-15 | INV.<br>A61K9/00<br>A61K9/08<br>A61K31/7088<br>A61K47/02 |
| X,D | BECK CHRISTINA ET AL: "Trimannose-coupled antimiR-21 for macrophage-targeted inhalation treatment of acute inflammatory lung damage", NATURE COMMUNICATIONS, vol. 14, no. 1, 28 July 2023 (2023-07-28), XP093255792, UK ISSN: 2041-1723, DOI: 10.1038/s41467-023-40185-1 Retrieved from the Internet: URL:https://www.nature.com/articles/s41467-023-40185-1> * abstract, figures 2-4 * ----- | 1-15 | TECHNICAL FIELDS SEARCHED (IPC)<br>A61K |
| X | US 2018/305691 A1 (BHAT BALKRISHEN [US] ET AL) 25 October 2018 (2018-10-25) * paragraph 478 * ----- | 1-15 | |

The present search report has been drawn up for all claims

1

| Place of search | Date of completion of the search | Examiner |
|---|---|---|
| The Hague | 27 March 2025 | Konter, Jörg |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

# ANNEX TO THE EUROPEAN SEARCH REPORT ON EUROPEAN PATENT APPLICATION NO. EP 24 21 2203

This annex lists the patent family members relating to the patent documents cited in the above-mentioned European search report.
The members are as contained in the European Patent Office EDP file on
The European Patent Office is in no way liable for these particulars which are merely given for the purpose of information.

27-03-2025

| Patent document cited in search report | Publication date | Patent family member(s) | Publication date |
|---|---|---|---|
| US 2018305691 A1 | 25-10-2018 | AR 090825 A1 | 10-12-2014 |
| | | AU 2013251696 A1 | 16-10-2014 |
| | | BR 112014026639 A2 | 27-06-2017 |
| | | CA 2869639 A1 | 31-10-2013 |
| | | CL 2014002844 A1 | 13-02-2015 |
| | | CL 2017001195 A1 | 26-01-2018 |
| | | CL 2019001878 A1 | 06-12-2019 |
| | | CN 104254607 A | 31-12-2014 |
| | | CO 7111303 A2 | 10-11-2014 |
| | | CR 20140524 A | 12-02-2015 |
| | | DO P2014000233 A | 31-12-2014 |
| | | EA 201491953 A1 | 30-06-2015 |
| | | EC SP14028612 A | 30-09-2015 |
| | | EP 2841579 A1 | 04-03-2015 |
| | | GT 201400221 A | 17-01-2018 |
| | | HK 1207115 A1 | 22-01-2016 |
| | | JP 6322189 B2 | 09-05-2018 |
| | | JP 2015519891 A | 16-07-2015 |
| | | KR 20150003846 A | 09-01-2015 |
| | | KR 20200064164 A | 05-06-2020 |
| | | KR 20220028183 A | 08-03-2022 |
| | | KR 20230037703 A | 16-03-2023 |
| | | MX 353562 B | 17-01-2018 |
| | | MY 173600 A | 08-02-2020 |
| | | NZ 630591 A | 24-02-2017 |
| | | PE 20142404 A1 | 02-02-2015 |
| | | PH 12014502387 A1 | 22-12-2014 |
| | | PH 12023550488 A1 | 24-06-2024 |
| | | SG 11201406931X A | 27-11-2014 |
| | | TW 201345918 A | 16-11-2013 |
| | | UA 117098 C2 | 25-06-2018 |
| | | US 2013289093 A1 | 31-10-2013 |
| | | US 2015218558 A1 | 06-08-2015 |
| | | US 2016244753 A1 | 25-08-2016 |
| | | US 2017240891 A1 | 24-08-2017 |
| | | US 2018305691 A1 | 25-10-2018 |
| | | WO 2013163258 A1 | 31-10-2013 |
| | | ZA 201407228 B | 31-05-2017 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2021205032 A


### Non-patent literature cited in the description


- **BECK et al.** *Nature Communications*, 2023, vol. 14, 4564